# EUROPEAN PATENT SPECIFICATION

(11) **EP 2 873 376 B1**
(45) Date of publication and mention of the grant of the patent: **20.02.2019**
(21) Application number: 13193180.0
(22) Date of filing: 15.11.2013
(51) Int. Cl.: A61B 17/02

(54) **Surgical retractor**
Chirurgischer Retraktor
Rétracteur chirurgical

(43) Date of publication of application: 20.05.2015
(73) Proprietor: University Of Dundee, Dundee DD1 4HN (GB)
(72) Inventor: Cuschieri, Alfred, St.Andrews, Fife KY16 9TY (GB); Mountain, Rodney, Fife KY15 4NB (GB); Steele, Peter, Dundee DD2 1BQ (GB); Martin, Duncan, Dundee DD2 IFD (GB); Coleman, Stuart, Dundee DD2 IFD (GB)
(74) Representative: Bobbert & Partner Patentanwälte PartmbB

(56) References cited:
- US-A1- 2005 171 405
- US-A1- 2005 215 865
- US-A1- 2007 232 864
- US-A1- 2007 235 038
- US-A1- 2008 269 564
- US-A1- 2011 028 797
- US-A1- 2013 082 157
- US-A1- 2013 137 934
- US-B1- 6 190 312
- US-B1- 6 468 207

## Description

The present invention relates to a surgical retractor. It relates further to a set comprising a surgical retractor and an optional surgical retractor frame for attaching the surgical retractor onto.

For keeping a wound open after an initial incision, a surgeon needs trained staff to retract tissue at his direction.

The document US 2008/0269564 A1 discloses a surgical system comprising a surgical retractor and an elastic member.

The document US 2007/0235038 A1 discloses a surgical system comprising a medical drape adapted to overlay the body of a patient.

The document US 2005/0215865 A1 discloses an apparatus for retracting flesh from a surgical site comprising a retractor support including a plurality of pegs and a flexible member having a plurality of apertures.

It is an object of the present invention to suggest devices for decreasing or eliminating the above mentioned need for assistance.

This object may be solved by the surgical retractor according to claim 1. It may also be solved by the set according to claim 6.

The surgical retractor according to the present invention is defined by the feature combination of claim 1. Accordingly, in one aspect of the invention, a surgical retractor (short: retractor) for use in performing surgery upon a portion of a patient's body having a surface contour is proposed, the retractor comprising a metal body having a first end for contact with the patient's tissue in order to retract the tissue by applying tension to it, and a second end opposite to the first end. The retractor also comprises an elastic tether (short: tether) attached to the second end or a second end portion of the metal body.

Herein, a surgical retractor frame is disclosed, which is configured for receiving or applying tension to at least one tether of a retractor. The retractor frame comprises or consists of at least one frame module or frame section. The frame module comprises at least one device for attaching at least one tether of the retractor to the retractor frame, or to parts thereof, or is interconnected with such a device.

The set according to the present invention is defined by the feature combination of claim 6. Accordingly, in yet another aspect of the present invention, a set is proposed which comprises at least one surgical retractor according to the present invention.

In all of the following exemplary embodiments, the use of the expressions 'may be' or 'may have' and so on, are to be understood synonymously with 'preferably is' or 'preferably has', respectively, and so on, and are intended to illustrate exemplary embodiments according to the present invention.

Embodiments according to the present invention can include one or more of the following features in arbitrary combinations.

In some exemplary embodiments according to the present invention, the metal body is manufactured from one or more sheets, strips or rods of metal.

In particular exemplary embodiments according to the present invention, a 'sheet' is a broad flat piece of material.

In certain exemplary embodiments according to the present invention, the metal body is manufactured from or consists of a sheet of metal only, or in its entirety, or from its first end to its second end.

In certain exemplary embodiments according to the present invention, the tether is manufactured from a sheet of elastic material.

In some exemplary embodiments according to the present invention, the tether is manufactured just from a sheet of material, or in its entirety or from the first end to the second end thereof, or consists thereof.

In certain exemplary embodiments according to the present invention, the tether comprises silicone or consists thereof.

Being manufactured from a sheet of material means, in particular embodiments according to the present invention, that the respective element (metal body, tether, or the like) has a flat shape or cross section. 'Flat' may mean that the width of the element or its cross section is several times its height. 'Flat' may mean having a broad level surface but little height or depth.

In some exemplary embodiments according to the present invention, at least parts of the metal body, or the entire metal body are malleable or bendable preferably just by hand and preferably without tools during use (i.e. at the bedside).

In certain exemplary embodiments according to the present invention, at least parts of the metal body or the entire metal body are made from stainless steel. Stainless steel is advantageously both ductile and biocompatible for short-term contact with the patient (no skin irritation or the like). Alternatively, parts of the metal body or the entire metal body may be formed from grey iron.

In some exemplary embodiments according to the present invention, the dimensions and/or the material properties of the metal body are chosen such that the metal body will not bend under a force of 5, 8 or 10 N as the retraction forces of the tether will be that high.

In certain exemplary embodiments according to the present invention, the dimensions and/or the material properties of the metal body are chosen such that the metal body will bend under a force of 10 N, 15 N, 20 N, 30 N, 40 N, or 50 N as the surgeon will be able to comfortably provide such bending forces by hand.

In some exemplary embodiments according to the present invention, the thickness of the metal body sheet is between 0.1 mm and 1 mm, in certain exemplary embodiments according to the present invention between 0.2 mm and 0.8 mm.

In some exemplary embodiments according to the present invention, the unbent dimensions of the metal body are, in a cross section, 0.4 mm x 8 mm, with a length of, preferably, 35 mm.

According to the present invention, the metal body - and optionally the tether - comprises an interconnecting device for interconnecting the tether with the metal body and for disconnecting them again.

In some exemplary embodiments according to the present invention, the interconnecting device is designed to be handled by the surgeon without the need to use tools.

According to the present invention, the interconnecting device comprises a through opening.

According to the present invention, the tether is attached by threading through a hole or feature on the metal body.

In certain exemplary embodiments according to the present invention, the elastic tether comprises, in a longitudinal direction thereof, a first end, a second end opposite the first end, and a through opening at the first end thereof. The through opening is at least as wide as the width of the tether at the second end.

In some exemplary embodiments according to the present invention, at least one of the metal body and the tether (i.e. either the metal body or the tether or both) have at least one of a flat cross section and a rectangular shape.

In particular exemplary embodiments according to the present invention, the metal body and/or the tether have a flat cross section and/or a rectangular shape along their entire length.

According to the present invention, the metal body and the tether have a through hole or opening so as to be connected with each other.

In some exemplary embodiments of the set according to the present invention, the retractor frame comprises at least two frame modules. The frame modules are configured to be interconnected with each other so as to form the retractor frame or be part thereof.

In certain exemplary embodiments of the set according to the present invention, the retractor frame is heavy enough so that the retractor frame's weight prevents slippage on the patient's body. This way, the retractor frame needs not to be attached or fixed to the surgical drapes.

In some exemplary embodiments of the set according to the present invention, the retractor frame weighs between 1 kg and 5 kg.

In particular exemplary embodiments of the set according to the present invention, the retractor frame comprises detachable weights that are attached to the retractor frame so as to increase its weight. These additional weights may be attached to the retractor frame at holes, attaching devices or attachment holes on the retractor frame.

In certain exemplary embodiments of the set according to the present invention, at least two frame modules each have elements for interconnecting the two frame modules with each other. Such interconnected frame modules can be adjacent or neighbouring (in relation to the whole retractor frame).

In some exemplary embodiments of the set according to the present invention, the elements for interconnecting are designed so as be interconnectable without need for further items, e.g., pins.

In certain exemplary embodiments of the set according to the present invention, the elements for interconnecting are designed so as be interconnectable without the need for tools.

In some exemplary embodiments of the set according to the present invention, the elements for interconnecting are designed so as be interconnectable such that the interconnected frame modules are not hingedly connected with each other.

In some exemplary embodiments of the set according to the present invention, the elements for interconnecting are plug-in elements or connections.

In some exemplary embodiments of the set according to the present invention, at least parts or sections of the retractor frame - or the entire frame - or some of, or all of the frame modules, or parts thereof, are malleable or bendable, preferably by hand during use.

In certain exemplary embodiments of the set according to the present invention, the retractor frame comprises at least one fixing device for fixing or attaching the retractor frame or parts thereof to surgical drapes.

In particular exemplary embodiments of the set according to the present invention, said at least one fixing device is a through hole or opening for a suture, for fixing the retractor frame or parts thereof to surgical drapes by sewing.

In some exemplary embodiments of the set according to the present invention, the retractor frame comprises in at least one section, or a part thereof, a malleable or bendable metal core and a flexible polymer that encompasses, coats or covers the core. The core may be rod-shaped or bar-shaped.

In certain exemplary embodiments according to the present invention, the set comprises at least two surgical retractors having different shapes, widths, hooks or sizes.

In some exemplary embodiments according to the present invention, the set comprises at least one retractor frame according to the present invention.

In certain exemplary embodiments according to the present invention, said at least one attaching device for attaching at least one tether of the retractor to the retractor frame, or to parts thereof, is a notch. In other embodiments, the attaching device is a slot. In yet other embodiments, the attaching device is a combination of a notch and a slot.

In some exemplary embodiments according to the present invention, the tether is a disposable or single-use tether.

In certain exemplary embodiments according to the present invention, one of the metal body and the tether (i.e. either the metal body or the tether or both) comprises a grip or a handle for the surgeon to grip and pull the retractor.

In some exemplary embodiments according to the present invention, the metal body comprises a hook with at least two teeth.

In certain exemplary embodiments according to the present invention, the metal body is pre-fabricated, bent or straight, and cannot be bent by hand.

In particular embodiments according to the present invention, the set comprises disposable or single-use retractors, or multi-use retractors, or both.

In particular exemplary embodiments according to the present invention, the retractor frame is sufficiently heavy so as to be placed onto the patient's surface without being prone to unintentional shifting.

In certain exemplary embodiments according to the present invention, the present invention may provide for one, some or all of the advantages stated herein.

In certain exemplary embodiments according to the present invention, the suggested devices may decrease or eliminate the need for assistance. The devices may replace some of the surgeon's assistant's work.

Also, in exemplary embodiments according to the present invention with at least a flat tether and/or a flat metal body, the retractor is less prone to account for ischaemia caused by the pressure applied by the retractor to the underlying tissue. The force transmitted by the retractor is simply widely or broadly distributed.

If, as in some exemplary embodiments according to the present invention, the metal body is manufactured from a sheet of metal, the metal body's torsional stiffness and bending stiffness is relatively high in a first direction, while its bending stiffness is relatively low in a second direction. Hence, the metal body is sufficiently stiff in the first direction while it can be adapted to need by the surgeon in the second direction.

In exemplary embodiments according to the present invention with bendable or malleable metal bodies or frame modules, the surgeon is free to adapt them into any shape he or she might need.

If tether and metal body are provided with an interconnecting device they may be separated from each other. This way, the metal body may be sterilized and thus reused and can be used several times. It may even be reused if the elastic tether, which might be non-reusable, has been damaged during preceding use. The interconnecting device allows for the use of another tether with the metal body.

If the attaching device is a slot, the tether may be pulled into the slot (which may be a tapering slot) or groove so that it becomes jammed in place. The surgeon would first adjust the tension/position of the retractor as required, and then pull the elastic tether into the nearest slot so that it becomes locked.

If the slot is tapered (wider at the top, narrow at the bottom), the tether can be raised in the slot so that it is able to be adjusted with little resistance, before being lowered again to lock its position.

Some interconnecting elements as described herein may allow for easy assembling of the retractor, without the need for tools or additional elements. The same may hold true for some of the elements for interconnecting adjacent frame modules described herein.

The retractor frame assembly based on individual frame modules which may be of different shapes could allow for the manufacture of a retractor frame that is really compatible with the specific need.

The flexible polymer may enhance the friction resistance between the retraction frame and the surgical drape it is laid on. This way, the flexible polymer may help to avoid slippage of the retractor frame on the drape. The fixing devices may serve the same function.

Other aspects, features, and advantages will be apparent from the description, figures, and claims. However, the present invention must not be understood to be limited to the exemplary embodiments shown in the figures. Identical reference numerals used in the figures depict identical or similar elements.
- **Fig. 1a, b, d**: show metal bodies of surgical retractors of three different exemplary embodiments according to the present invention;
- **Fig. 2**: shows an exemplary surgical retractor according to the present invention;
- **Fig. 3a, b, c**: show metal bodies of exemplary surgical retractors according to the present invention in different sizes;
- **Fig. 4**: shows a surgical retractor according to another example not forming part of the present invention;
- **Fig. 5**: shows yet another exemplary embodiment of the present invention, in which the metal body of the surgical retractor comprises a grip-flap;
- **Fig. 6**: shows a single frame module of a retractor frame in an exemplary embodiment of the set according to the present invention;
- **Fig. 7a, b, c**: show retractor frames of different exemplary embodiments of the set according to the present invention;
- **Fig. 8**: shows a first exemplary configuration for the retractor frame according to an embodiment of the set according to the present invention;
- **Fig. 9**: shows a second exemplary configuration for the retractor frame according to an embodiment of the set according to the present invention;
- **Fig. 10**: shows a cross section of a retractor frame according to an exemplary embodiment of the set according to the present invention;
- **Fig. 11**: shows, in a plan view, part of a frame module of the retractor frame in an exemplary embodiment of the set according to the present invention; and
- **Fig. 12**: shows, in a perspective view, the frame module of Fig. 11.

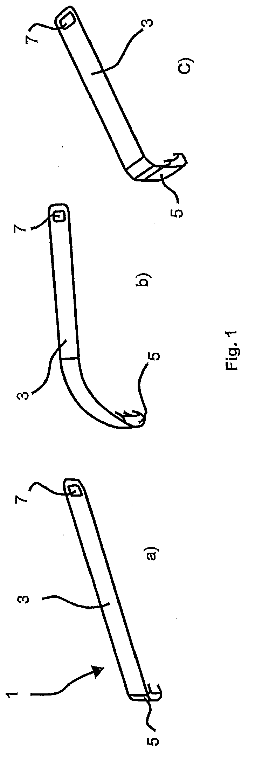

**Figs. 1a** to **1d** each show parts of surgical retractors 1 (short: retractors 1), in different shapes. What is shown are the metals bodies 3 of the retractors 1. Not shown in Figs. 1a to 1d are the elastic tethers (short: tethers) that also form part of the retractors 1.

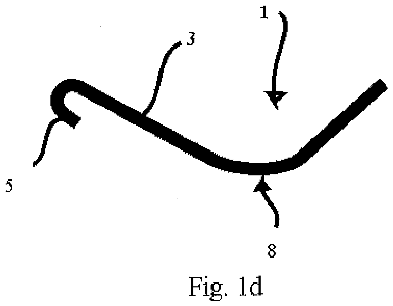

As can be seen from Figs. 1a to 1d, the retractors 1 are all made from a flat metal sheet. Also, in the exemplary embodiments shown in Figs. 1a to 1d all comprise a hook 5 at a first end thereof and a through hole or opening 7 (short: opening 7, not shown in Fig. 1d) at a second end thereof. The openings 7 are intended for interconnecting the elastic tethers not shown in Figs. 1a to 1d with the metal bodies 3.

Whereas the metal body 3 of Fig. 1a is only bent at its first end to assume a hook shape, it is straight and plain in all other parts.

In contrast, the metal body 3 shown in Fig. 1b is smoothly bent about its first end such that its metal body 3 is partly plain and partly bent.

Fig. 1c shows yet another bending of the metal body 3. When compared to the metal body 3 of Fig. 1b, the plain part is longer, and the bending angle is more acute than the one shown in Fig. 1b.

Fig. 1d shows yet another bending of the metal body 3. When compared to the metal body 3 of Fig. 1b or 1c, the metal body 3 is not bent once, but twice and in opposite directions (or: once concave and once convex). This way, the retractor 1 forms a heel 8 or convex curved surface which may be pushed against the sound tissue and used as a fulcrum for raising the hook 5 up, in order to open the wound further.

As stated supra, bending of the metal body 3 is, in certain exemplary embodiments according to the present invention, left to the surgeon's discretion. In those embodiments he or she may choose a preferred shape for the metal body 3 which best meets actual need and he or she may bend the metal body 3 accordingly by hand.

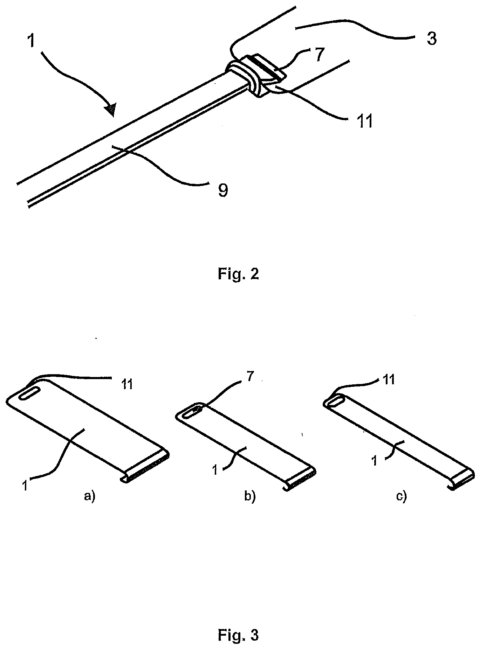

**Fig. 2** shows a surgical retractor 1 according to the present invention comprising an elastic tether 9 (short: tether 9).

As can be seen in Fig. 2, a first end of the tether 9 has obviously been passed through the opening 7 of the metal body 3 and also through an opening provided in a second end portion of the tether 9 so as to form a closed loop around a strut 11 of the metal body 3 formed by the opening 7. When pulling the tether 9, it is contracted around the strut 11.

The opening 7 and the closed loop are only one example of an interconnecting device for attaching the tether 9 to, detaching it from, and re-attaching it to, the metal body 3.

**Fig. 3a, 3b,** and **3c** show metal bodies 3 of surgical retractors 1 according to the present invention in different sizes.

The metal bodies 3 all differ from each other in width only. However, they can also be provided in different shapes, different dimensions, with differently formed hooks, and the like. If provided together, a quantity of metal bodies 3 (plus their tethers 9 optionally) like the ones shown in Figs. 3a to 3c can be called a set according to the present invention.

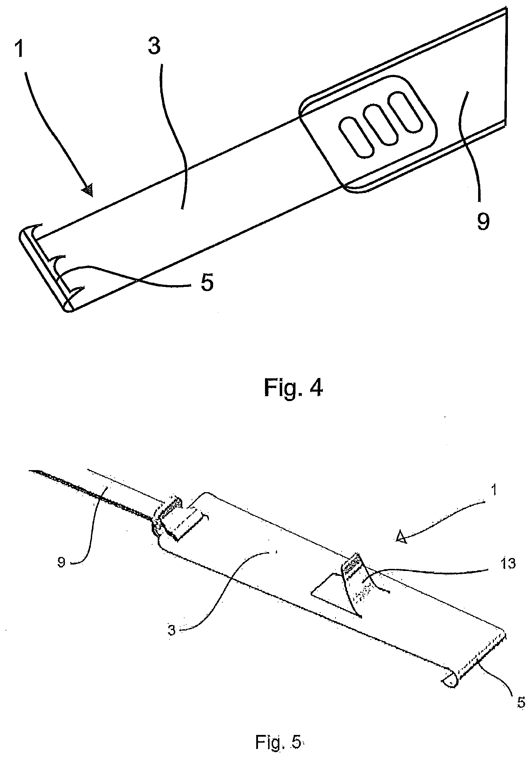

**Fig. 4** shows a surgical retractor 1 according to another example not forming part of the present invention. In contrast with how the metal body 3 is attached to the tether 9 (or vice versa) in Fig. 2, the tether 9 is moulded onto, or over, the second end of metal body 3. Hence, in the embodiment of Fig. 4, parts of the metal body 3 are retained inside of the tether 9. In such embodiments, the retractor 1 can be called an integral instrument.

**Fig. 5** shows yet another embodiment of the present invention in which the metal body 3 of the retractor 1 comprises a grip-flap 13. The grip-flap 13 serves the surgeon as a handle or grip upon positioning, re-positioning, tensioning, or any manoeuvre with respect to the retractor 1.

In the example of Fig. 5, the grip-flap 13 is generated by punching the metal body 3 and bending the so generated flap upwards. This appears to be the cheapest and easiest way to provide such a handle for the surgeon. However, the present invention also envisages and encompasses several optional handles or grips, which may be generated as described herein or in any other way. Also, the grip does not necessarily have to be on the metal body 3. It may instead be attached to the tether 9.

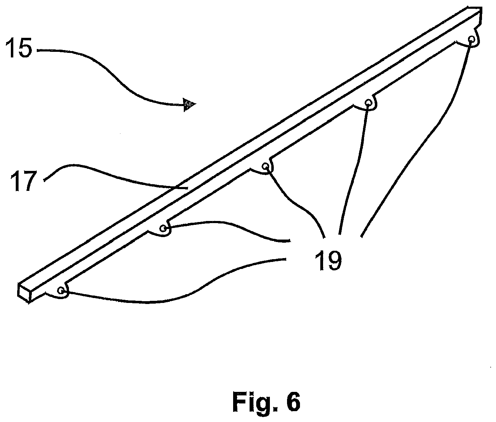

**Fig. 6** shows a retractor or tether frame 15 in a first embodiment of the set according to the present invention. In this embodiment, the retractor frame 15 consists of a single frame module 17.

The frame module 17 optionally comprises a number of evenly or unevenly spaced openings or holes 19 to aid suturing of the retractor frame 15 to surgical drapes (not shown in the figures), in order to keep the retractor frame 15 in a predetermined position with regard to the wound. The holes 19 are only one exemplary embodiment of fixing devices for fixing the retractor frame, or parts thereof, at surgical drapes.

In the example of Fig. 6, both the retractor frame 15, and hence the single frame module 17, are straight. However, they may be provided in any shape desired by the surgeon. Also, since in a number of exemplary embodiments of the set according to the present invention the retractor frame 15 is either freely, or at least within a certain range, malleable or bendable by the surgeon, the retractor frame 15 may bend into certain shapes according to need.

In Fig. 6, elements for interconnecting have not been shown for illustrational reasons only.

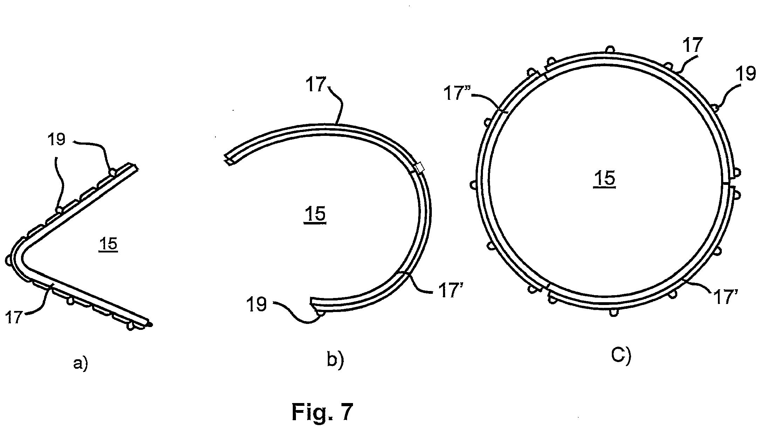

**Fig. 7a, 7b,** and **7c** show retractor frames 15 in different embodiments of the set according to the present invention.

As can be seen in Fig. 7a which shows the retractor frame 15 consisting of the single frame module 17 of Fig. 6, the shape of the retractor frame 15 can be amended by bending one or more of the frame modules 17 used.

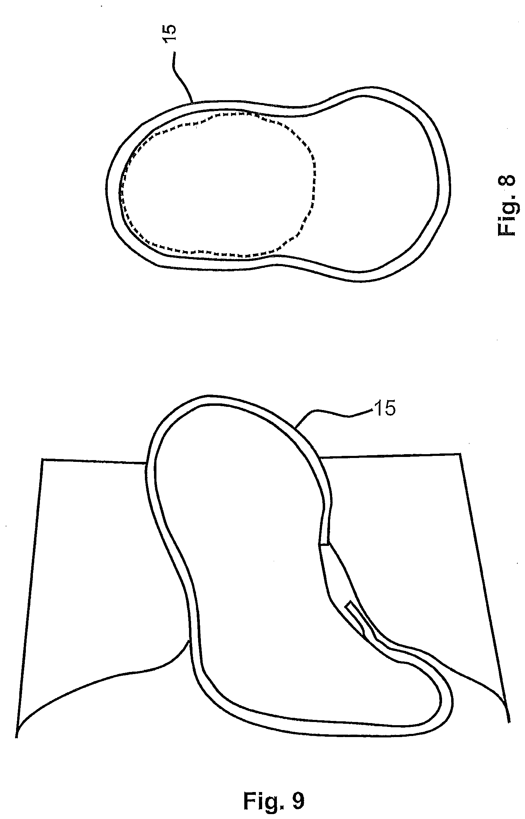

As can be seen in Figs. 7b and 7c, a desired shape of the retractor frame 15 can be alternatively or additionally achieved by using two (Fig. 7b) or more (Fig. 7c) tether frame modules 17, 17', 17", e.g. straight ones like the one shown in Figs. 6 and 7a (and possibly also by bending these) or by any other shape of frame module, such as curved. This way, almost any desired retractor frame shape may be provided, not just a U-shaped or an annular one as shown in Figs. 7b and 7c. Examples of further retractor frame shapes are shown in Figs. 8 and 9.

**Fig. 8** and **9** show two exemplary configurations for exemplary retractor frames 15 in use according to embodiments according to the set of the present invention.

In Fig. 8, the retractor frame 15 is used for head surgery, in Fig. 9 for neck surgery. In any case, the retractor frame 15 may advantageously be shaped so as to grip the patient's anatomy without additional fixation.

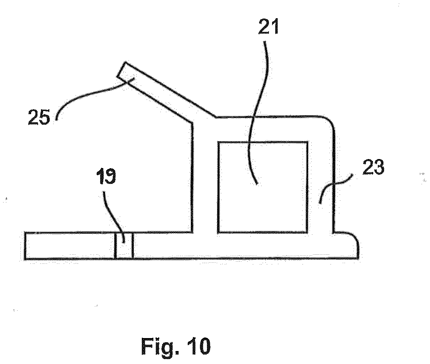

**Fig. 10** shows a cross section of a retractor frame 15 according to an exemplary embodiment of the set of the present invention.

The retractor frame 15 comprises, in at least one section thereof, a metal core 21 which is coated or covered by a flexible polymer 23. The metal core 21 contributes to the stability of the retractor frame 15 with regards to avoiding unintended bending thereof. Further, the additional weight provided by the metal core 21 may help to stabilise or keep the retractor frame 15 on the patient's surface, which in certain applications may allow dispose of the need to attach the retractor frame 15 to the surgical drapes at all.

As can be derived from Fig. 10, the metal core 21 or the flexible polymer 23 may have attaching devices 25 for attaching one or more tethers 9 of the surgical retractor 1 to the retractor frame 15 or parts thereof. In the example of Fig. 10, theses devices are embodied as protrusions or as slots or tapering. The protrusions or slots or tapering slots may be 'V'-shaped. They may, however, also have any other shape. For enhanced stability, the holes 19, if provided, may be provided at the same side of the metal core 21 as the attaching devices 25 for attaching the tether 9.

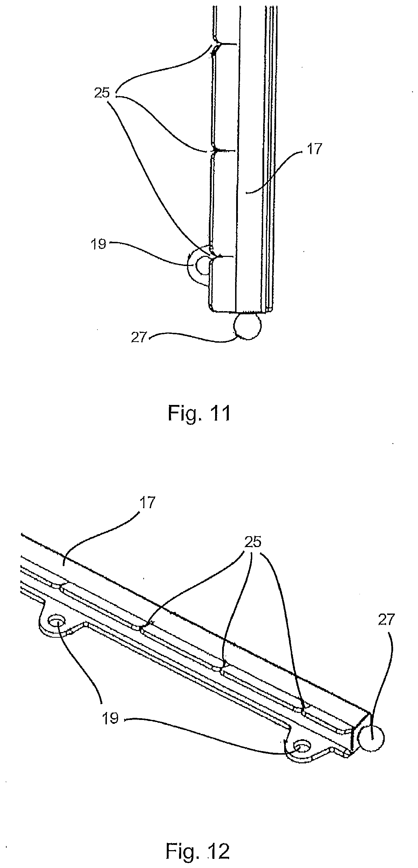

**Fig. 11** shows in a plan view part of a frame module 17 of the retractor frame 15 in one embodiment of the set according to the present invention. The frame module 17 comprises an element 27, here optionally designed as a plug-in element, for interconnecting adjacent frame modules (17, 17', 17"). The element 27 shown in Fig. 11 would fit into a corresponding hole or reception of an adjacent frame module not shown in the figures. This way, chains of frame modules can be created with ease. No tools are required to do so.

As can be seen in Fig. 11, as in a number of exemplary embodiments of the set according to the present invention, the attaching devices 25 are designed as notches.

**Fig. 12** shows the frame module 17 of Fig. 11 in a perspective view.

Again, it is noted that the figures relate to examples showing how exemplary embodiments according to the invention may be carried out. They are not to be understood as limiting the invention.

Also, the embodiments according to the invention may comprise one or more features as set forth supra which may be combined with any feature disclosed somewhere else in the present specification wherever such combination is technically possible from the perspective of the skilled person.

### Reference numerals

- 1: surgical retractor (short: retractor)
- 3: metal body
- 5: hook
- 7: through opening
- 8: heel
- 9: elastic tether (short: tether)
- 11: strut
- 13: grip-flap
- 15: retractor frame or tether frame
- 17, 17', 17": frame modules
- 19: hole
- 21: core
- 23: flexible polymer
- 25: attaching device for attaching a tether of the surgical retractor to the retractor frame
- 27: element for interconnecting adjacent frame modules

## Claims

1. A surgical retractor (1) for use in performing surgery upon a portion of a patient's body, comprising
- a metal body (3) comprising a first end for applying tension onto the patient's tissue in order to retract the tissue, and a second end opposite to the first end;
- an elastic tether (9) attached to the metal body (3) at the second end or a second end portion of the metal body (3),
- wherein the metal body (3) comprises an interconnecting device for detachably interconnecting the tether (9) with, and disconnecting it from, the metal body (3),
**characterised in that**
- the interconnecting device comprises a through opening (7); and wherein
- the elastic tether (9) comprises in a longitudinal direction thereof a first end and a second end portion,
wherein the first end of the elastic tether (9) is passed through the opening (7) of the metal body (3) and also through an opening provided in the second end portion of the tether (9) so as to form a closed loop around a strut (11) of the metal body (3) formed by the opening (7) of the metal body (3).

2. The retractor (1) according to claim 1, wherein
- the metal body (3) is manufactured from a sheet of metal.

3. The retractor (1) according to claim 1 or 2, wherein
- the tether (9) is manufactured from a sheet of elastic material.

4. The retractor (1) according to any one of the preceding claims, wherein
- at least parts of the metal body (3) are malleable or bendable during use.

5. The retractor (1) according to any one of the preceding claims, wherein
- at least one of the metal body (3) and the tether (9) have at least one of a flat cross section and a rectangular shape.

6. A set comprising at least one surgical retractor (1) according to at least one of claims 1 to 5.

7. The set according to claim 6, comprising at least two surgical retractors (1) having different shapes, widths, hooks or sizes.

8. The set according to claim 6 or 7, comprising at least one retractor frame (15) wherein the surgical retractor frame (15) is configured for receiving or tensioning at least one tether (9) of the surgical retractor (1), the retractor frame (15) comprising
- at least one frame module or frame section (17, 17', 17") having at least one attaching device (25) for attaching at least one tether (9) of the surgical retractor (1) to the retractor frame (15), or to parts thereof, or being interconnected with such an attaching device (25).

9. The set according to claim 8, the retractor frame (15) comprising at least two frame modules (17, 17', 17") configured to be interconnected with each other so as to form the retractor frame (15).

10. The set according to claim 8 or 9, wherein at least two frame modules (17, 17', 17") each have elements (27), in particular plug-in elements, for interconnecting adjacent frame modules (17, 17', 17") with each other.

11. The set according to any of claims 8 to 10, wherein at least parts of the retractor frame (15), or some or all of the frame modules (17, 17', 17"), or parts thereof, are malleable or bendable during use.

12. The set according to any of claims 8 to 11, the retractor frame (15) comprising at least one fixing device (19) for attaching the retractor frame (15) or parts thereof to surgical drapes.

13. The set according to any of claims 8 to 12, the retractor frame (15) comprising in at least one section, or a part thereof, a malleable or bendable metal core (21) and a flexible polymer (23) for at least one of encompassing, coating and covering the core (21).

## Patentansprüche

1. Chirurgischer Retraktor (1) zur Verwendung bei chirurgischen Eingriffen an einem Abschnitt des Körpers eines Patienten, wobei der chirurgische Retraktor (1) aufweist:
- einen Metallkörper (3), welcher ein erstes Ende zum Aufbringen von Spannung auf ein Gewebe des Patienten zum Zurückziehen des Gewebes umfasst sowie ein zweites Ende, welches gegenüber dem ersten Ende vorgesehen ist;
- einen elastischen Zugabschnitt (9), welcher mit dem zweiten Ende oder einem zweiten Endabschnitt des Metallkörpers (3) mit Letzterem verbunden ist;
- wobei der Metallkörper (3) eine Verbindungseinrichtung zum lösbaren Verbinden des Zugabschnitts (9) mit dem Metallkörper (3) und zu seinem Lösen vom Metallkörper (3) aufweist,
wobei
- die Verbindungseinrichtung eine Durchgangsöffnung (7) aufweist; und wobei
- der elastische Zugabschnitt (9) in seiner Längsrichtung einen ersten Endabschnitt und einen zweiten Endabschnitt aufweist;
wobei das erste Ende des elastischen Zugabschnitts (9) durch die Öffnung (7) der Verbindungseinrichtung geführt ist,
**dadurch gekennzeichnet, dass** das erste Ende des elastischen Zugabschnitts (9) auch durch eine Öffnung geführt ist, die im zweiten Endabschnitt des Zugabschnitts (9) vorgesehen ist, um eine geschlossene Schlaufe um einen Steg (11) des Metallkörpers (3) zu bilden, der durch die Öffnung (7) der Verbindungseinrichtung ausgebildet ist.

2. Chirurgischer Retraktor (1) nach Anspruch 1, wobei
- der Metallkörper (3) aus einem Metallblech hergestellt ist.

3. Chirurgischer Retraktor (1) nach Anspruch 1 oder 2, wobei
- der Zugabschnitt (9) aus einer Bahn elastischen Materials hergestellt ist.

4. Chirurgischer Retraktor (1) nach einem der vorangegangenen Ansprüche, wobei
- zumindest Teile des Metallkörpers (3) während ihres Gebrauchs verformbar oder biegsam sind.

5. Chirurgischer Retraktor (1) nach einem der vorangegangenen Ansprüche, wobei
- zumindest der Metallkörper (3) und/oder der Zugabschnitt (9) einen flachen Querschnitt und/oder eine rechteckige Form aufweisen.

6. Set, welches wenigstens einen chirurgischen Retraktor (1) nach einem der Ansprüche 1 bis 5 aufweist.

7. Set nach Anspruch 6, welches wenigstens zwei chirurgische Retraktoren (1) mit unterschiedlichen Formen, Breiten, Haken oder Größen aufweist.

8. Set nach Anspruch 6 oder 7, welches wenigstens einen Retraktorrahmen (15) aufweist, wobei der
Retraktorrahmen (15) ausgestaltet ist, um wenigstens einen Zugabschnitt (9) des chirurgischen Retraktors (1) aufzunehmen oder zu spannen, wobei der
Retraktorrahmen (15) aufweist:
- zumindest ein Rahmenmodul oder einen Rahmenabschnitt (17, 17', 17") mit wenigstens einer Befestigungseinrichtung (25) zum Befestigen wenigstens eines Zugabschnitts (9) des chirurgischen Retraktors (1) am Retraktorrahmen (15), oder an Teilen hiervon, oder um mit einer solchen Befestigungseinrichtung (25) verbunden zu werden.

9. Set nach Anspruch 8, wobei der Retraktorrahmen (15) wenigstens zwei Rahmenmodule (17, 17', 17") aufweist, welche ausgestaltet sind, um miteinander zum Ausbilden des Retraktorrahmens (15) verbunden zu werden.

10. Set nach Anspruch 8 oder 9, wobei wenigstens zwei Rahmenmodule (17, 17', 17") jeweils Elemente (27) aufweisen, insbesondere Einsteck-Elemente, zum Verbinden benachbarter Rahmenmodule (17, 17', 17") miteinander.

11. Set nach einem der Ansprüche 8 bis 10, wobei wenigstens Teile des Retraktorrahmens (15), oder einige oder alle der Rahmenmodule (17, 17', 17"), oder Teile hiervon, während ihres Gebrauchs verformbar oder biegsam sind.

12. Set nach einem der Ansprüche 8 bis 11, wobei der Retraktorrahmen (15) wenigstens eine Fixiereinrichung (19) zum Befestigen des Retraktorrahmens (15) oder Teile hiervon an chirurgischen Tüchern aufweist.

13. Set nach einem der Ansprüche 8 bis 12, wobei der Retraktorrahmen (15) in wenigstens einem Abschnitt oder Teil hiervon einen verformbaren oder biegsamen Metallkern (21) und ein flexibles Polymer (23) zum Umgeben, Beschichten und/oder Bedecken des Metallkerns (21) aufweist.

## Revendications

1. Un écarteur chirurgical (1), destiné à être utilisé lors d'une intervention chirurgicale sur une partie du corps d'un patient, comprenant:
- un corps métallique (3) comprenant une première extrémité pour appliquer une tension sur le tissu d'un patient afin de rétracter le tissu, et une seconde extrémité prévue à l'opposé de la première extrémité;
- une attache élastique (9) fixée au corps métallique (3) au niveau de la seconde extrémité ou au niveau d'une seconde section terminale du corps métallique (3),
- où le corps métallique (3) comprend un dispositif d'interconnexion pour connecter l'attache (9) de manière amovible et la déconnecter du corps métallique (3).
où
- le dispositif d'interconnexion comprend un orifice de passage (7); et où
- l'attache élastique (9) comprend, dans son sens longitudinal, une première et une seconde section terminale;
où la première extrémité de l'attache élastique (9) est guidée à travers l'orifice (7) du dispositif d'interconnexion,
**caractérisé en ce que** la première extrémité de l'attache élastique (9) est également guidée à travers un orifice présent dans la seconde section terminale de l'attache (9), de manière à créer une boucle fermée autour d'un montant (11) du corps métallique (3) formé par l'orifice (7) du dispositif d'interconnexion.

2. L'écarteur chirurgical (1) selon la première revendication, où
- le corps métallique (3) est fabriqué à partir d'une feuille métallique.

3. L'écarteur chirurgical (1) selon la revendication 1 ou 2, où
- l'attache (9) est fabriquée à partir d'une feuille de matériau élastique.

4. L'écarteur chirurgical (1) selon l'une quelconque des revendications précédentes, où
- au moins certaines parties du corps métallique (3) sont déformables ou flexibles lors de leur utilisation.

5. L'écarteur chirurgical (1) selon l'une quelconque des revendications précédentes, où
- le corps métallique (3) et/ou l'attache (9) présente/présentent au moins une section transversale plane et/ou une forme rectangulaire.

6. Un kit comprenant au moins un écarteur chirurgical (1) selon au moins l'une quelconque des revendications 1 à 5.

7. Le kit selon la revendication 6, comprenant au moins deux écarteurs chirurgicaux (1) de formes, largeurs, crochets ou de tailles différents.

8. Le kit selon la revendication 6 ou 7, comprenant au moins un support d'écarteur (15), où le cadre d'écarteur chirurgical (15) est conçu pour recevoir ou mettre sous tension au moins une attache (9) de l'écarteur chirurgical (1), le cadre d'écarteur (15) comprenant:
- au moins un module de support ou une section de support (17, 17', 17") ayant au moins un dispositif de fixation (25) pour fixer au moins une attache (9) de l'écarteur chirurgical (1) au support de l'écarteur (15), ou à des parties de celui-ci, ou étant connecté à un tel dispositif de fixation (25).

9. Le kit selon la revendication 8, où le support de l'écarteur (15) comprend au moins deux modules de support (17, 17', 17") conçus pour être connectés ensemble de façon à former le support de l'écarteur (15).

10. Le kit selon la revendication 8 ou 9, où au moins deux modules de support (17, 17', 17") comprennent chacun des éléments (27), en particulier des éléments enfichables, pour connecter des modules de support adjacents (17, 17', 17") les uns aux autres.

11. Le kit selon l'une quelconque des revendications 8 à 10, où au moins certaines parties du support de l'écarteur (15), ou certains ou la totalité des modules de support (17, 17' , 17"), ou des parties de ceux-ci, sont déformables ou flexibles lors de leur utilisation.

12. Le kit selon l'une quelconque des revendications 8 à 11, où le support de l'écarteur (15) comprend au moins un dispositif de fixation (19) pour fixer le support de l'écarteur (15) ou des parties de celui-ci à des drapés chirurgicaux.

13. Le kit selon l'une quelconque des revendications 8 à 12, où le support de l'écarteur (15) comprend dans au moins une section, ou dans une partie de celle-ci, un noyau métallique (21) déformable ou flexible ainsi qu'un polymère souple (23) pour au moins englober, enrober et recouvrir le noyau métallique (21).
